# EUROPEAN PATENT APPLICATION

(11) **EP 4 389 745 A1**
(43) Date of publication of application: **26.06.2024**
(21) Application number: 22815335.9
(22) Date of filing: 02.06.2022
(51) Int. Cl.: C07D 471/00, C07D 487/00, C07D 495/00, C07D 497/00, C07D 239/00, C07D 241/36, A61K 31/50, A61K 31/495, A61P 31/18

(54) **PYRIDONE COMPOUND HAVING INTEGRASE INHIBITORY ACTIVITY AND PHARMACEUTICAL USE THEREOF**

(30) Priority: 03.06.2021 CN 202110619370; 28.01.2022 CN 202210104300
(71) Applicant: Jiangsu Hengrui Pharmaceuticals Co., Ltd., Lianyungang, Jiangsu 222047 (CN); Shanghai Senhui Medicine Co., Ltd., Shanghai 201203 (CN); Retrolead (Shanghai) Biopharma Co., Ltd., Shanghai 200131 (CN)
(72) Inventor: ZHU, Lingjian, Shanghai 201203 (CN); HONG, Min, Shanghai 201203 (CN); TAO, Weifeng, Lianyungang, Jiangsu 222047 (CN); WU, Daize, Lianyungang, Jiangsu 222047 (CN); PAN, Xiaoyu, Lianyungang, Jiangsu 222047 (CN); GAO, Lu, Shanghai 200131 (CN); HUANG, Jian, Shanghai 201203 (CN)
(74) Representative: Potter Clarkson
(86) International application number: PCT/CN2022/096750
(87) International publication number: WO 2022/253294

(57) **Abstract**

The present disclosure provides a pyridone compound having integrase inhibitory activity and a pharmaceutical use thereof. Specifically, the present disclosure provides a compound having a structure shown as formula I or a pharmaceutically acceptable salt thereof, which can be used to treat human immunodeficiency (HIV) infection.

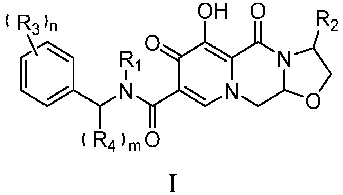

## Description

The present disclosure belongs to the field of medicine, and relates to a pyridone compound having integrase inhibitory activity and a pharmaceutical use thereof.

### BACKGROUND OF THE INVENTION

Human immunodeficiency virus (HIV) is also known as human immunodeficiency virus. It is the most structurally complex retrovirus known, and includes Acquired Immune Deficiency Syndrome (AIDS) virus. When entering the AIDS stage, AIDS virus infection will cause various opportunistic infections and tumors. These complications will have an impact on people's health, and may even be life-threatening. Therefore, AIDS virus causes panic among people.

Human immunodeficiency virus type 1 (HIV-1) encodes three enzymes required for viral replication: reverse transcriptase, protease, and integrase. Although drugs targeting reverse transcriptase and protease are widely used and have been shown to be effective, particularly when applied in combination, toxicity and development of resistant strains limit their use.

On the other hand, due to the frequent emergence of drug-resistant viruses, multidrug combination therapy is effective in the treatment of AIDS. Two of reverse transcriptase inhibitors and protease inhibitors are used clinically as anti-HIV drugs, but drugs with the same mechanism often exhibit cross-resistance or only exhibit additive efficacy. Therefore, the development of anti-HIV drugs with different mechanisms is required.

Under this situation, integrase inhibitors, as anti-HIV drugs with new mechanisms, have attracted much attention (WO2006116764A1 and WO2013054862A1). HIV integrase inhibitors are anti-HIV/AIDS drugs with a new mechanism. They can be administered in combination with additional antiretroviral drugs to effectively treat HIV infection, and are not prone to drug resistance in clinical.

Cocktail therapy consisting of HIV integrase inhibitors and marketed reverse transcriptase inhibitors, protease inhibitors and fusion inhibitors can solve the problems of single drug resistance and predisposing cross-resistance. It will significantly improve the therapeutic effect of existing HIV drugs, and become a new treatment approach and option.

HIV integrase inhibitor drugs currently approved for marketing and entering clinical use include: raltegravir, elvitegravir, dolutegravir, bictegravir, cabotegravir and the fixed-dose combination finished pharmaceutical product Stribild.

### SUMMARY OF THE INVENTION

The present disclosure provides a compound of formula I or a pharmaceutically acceptable salt thereof, 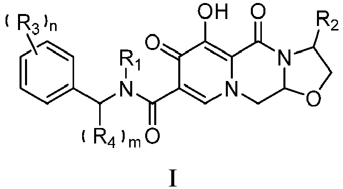 wherein,
R₁ is selected from the group consisting of hydrogen, C₁₋₆ alkyl, C₃₋₆ cycloalkyl, 3 to 6 membered heterocyclyl and hydroxy, the C₁₋₆ alkyl, C₃₋₆ cycloalkyl and 3 to 6 membered heterocyclyl are each optionally substituted by one to three R₅ₐ;
R₂ is selected from the group consisting of hydrogen, halogen, C₁₋₆ alkyl substituted by 1 to 3 halogen, C₃₋₆ cycloalkyl, 3 to 6 membered heterocyclyl, hydroxy, methylenecyclopropyl and oxo, the C₃₋₆ cycloalkyl, 3 to 6 membered heterocyclyl and methylenecyclopropyl are each optionally substituted by one to three R_{5b};
each R₃ is independently selected from the group consisting of halogen, cyano, amino, nitro, C₁₋₆ alkyl, C₃₋₆ cycloalkyl, 3 to 6 membered heterocyclyl and hydroxy, the C₁₋₆ alkyl, C₃₋₆ cycloalkyl and 3 to 6 membered heterocyclyl are each optionally substituted by one to three R_{5c};
n is an integer selected from 0 to 4;
each R₄ is independently selected from the group consisting of halogen, cyano, amino, nitro, C₁₋₆ alkyl, C₃₋₆ cycloalkyl, 3 to 6 membered heterocyclyl, hydroxy and oxo, the C₁₋₆ alkyl, C₃₋₆ cycloalkyl and 3 to 6 membered heterocyclyl are each optionally substituted by one to three R_{5d};
m is an integer from 0 to 2;
R₅ₐ, R_{5b}, R_{5c} and R_{5d} are each independently selected from the group consisting of halogen, C₁₋₆ alkyl, C₃₋₆ cycloalkyl, 3 to 6 membered heterocyclyl, hydroxy, cyano, amino and nitro.

In some embodiments, the compound of formula I can be: 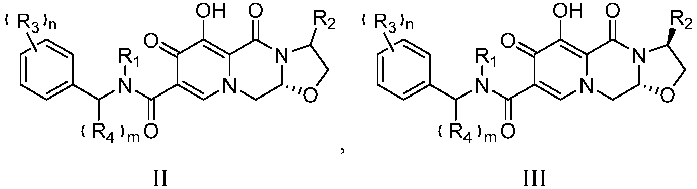
In some embodiments, in the compound of formula I, formula II or formula III or the pharmaceutically acceptable salt thereof, R₁ is selected from the group consisting of hydrogen, C₁₋₆ alkyl, C₃₋₆ cycloalkyl and hydroxy, the C₁₋₆ alkyl and C₃₋₆ cycloalkyl are each optionally substituted by one to three R₅ₐ;
R₅ₐ is selected from the group consisting of halogen, C₁₋₆ alkyl, C₃₋₆ cycloalkyl, hydroxy, cyano, amino and nitro;
preferably, R₁ is selected from the group consisting of hydrogen, C₁₋₆ alkyl, C₃₋₆ cycloalkyl and hydroxy, the C₁₋₆ alkyl and C₃₋₆ cycloalkyl are each optionally substituted by one to three R₅ₐ; R₅ₐ is selected from the group consisting of halogen, methyl, ethyl, isopropyl, cyclopropyl, *n*-propyl, hydroxy, cyano, amino and nitro.

In some embodiments, in the compound of formula I, formula II or formula III or the pharmaceutically acceptable salt thereof, each R₄ is independently selected from the group consisting of halogen, cyano, amino, nitro, C₁₋₆ alkyl, C₃₋₆ cycloalkyl, hydroxy and oxo, the C₁₋₆ alkyl and C₃₋₆ cycloalkyl are each optionally substituted by one to three R_{5d};
each R_{5d} is independently selected from the group consisting of halogen, C₁₋₆ alkyl, C₃₋₆ cycloalkyl, hydroxy, cyano, amino and nitro;
preferably, each R₄ is independently selected from the group consisting of halogen, cyano, amino, nitro, C₁₋₆ alkyl, C₃₋₆ cycloalkyl, hydroxy and oxo.

In some embodiments, in the compound of formula I, formula II or formula III or the pharmaceutically acceptable salt thereof, m is an integer from 0 to 2; preferably, m is an integer from 0 to 1; and more preferably, m is 0.

In some embodiments, in the compound of formula I, formula II or formula III or the pharmaceutically acceptable salt thereof, each R₃ is independently selected from the group consisting of halogen, cyano, amino, nitro, C₁₋₆ alkyl, C₃₋₆ cycloalkyl and hydroxy, the C₁₋₆ alkyl and C₃₋₆ cycloalkyl are each optionally substituted by one to three R_{5c};
n is an integer selected from 0 to 4;
each R_{5c} is independently selected from the group consisting of halogen, C₁₋₆ alkyl, C₃₋₆ cycloalkyl, hydroxy, cyano, amino and nitro;
preferably, each R₃ is independently selected from the group consisting of halogen, cyano, amino, nitro, C₁₋₆ alkyl, C₃₋₆ cycloalkyl and hydroxy, the C₁₋₆ alkyl and C₃₋₆ cycloalkyl are each optionally substituted by one to three R_{5c};
n is an integer selected from 0 to 2;
each R_{5c} is independently selected from the group consisting of halogen, methyl, ethyl, cyclopropyl, isopropyl, hydroxy, cyano, amino and nitro;
more preferably, each R₃ is independently selected from the group consisting of halogen, cyano, amino, nitro, methyl, ethyl, cyclopropyl, isopropyl and hydroxy, the methyl, ethyl, cyclopropyl and isopropyl are each optionally substituted by one to three R_{5c};
n is an integer selected from 0 to 2;
each R_{5c} is independently selected from the group consisting of halogen, methyl, ethyl, cyclopropyl, isopropyl, hydroxy, cyano, amino and nitro;
most preferably, each R₃ is independently selected from halogen; n is an integer selected from 0 to 2.

The present disclosure also provides a compound of formula IV or a pharmaceutically acceptable salt thereof, 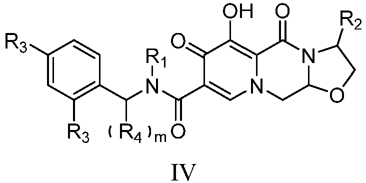 R₁, R₂, R₃, R₄ and m are as defined in formula I.

The present disclosure also provides a compound of formula V or a pharmaceutically acceptable salt thereof,

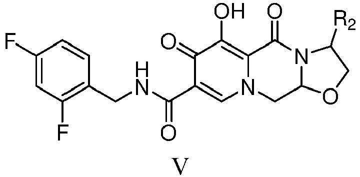

R₂ is as defined in formula I.
In some embodiments, in the compound of formula I, formula II, formula III, formula IV or formula V or the pharmaceutically acceptable salt thereof, R₂ is selected from the group consisting of hydrogen, halogen, C₁₋₆ alkyl substituted by 1 to 3 halogen, C₃₋₆ cycloalkyl, hydroxy, methylenecyclopropyl and oxo, the C₃₋₆ cycloalkyl and methylenecyclopropyl are each optionally substituted by one to three R_{5b};
preferably, R₂ is selected from the group consisting of hydrogen, halogen, C₁₋₆ alkyl substituted by 1 to 3 halogen, and C₃₋₆ cycloalkyl, the C₃₋₆ cycloalkyl is optionally substituted by one to three R_{5b};
more preferably, R₂ is selected from the group consisting of methyl substituted by 1 to 3 fluorine, and cyclopropyl, the cyclopropyl is optionally substituted by one to three halogen;
most preferably, R₂ is selected from the group consisting of monofluoromethyl and cyclopropyl.

In some embodiments, in the compound of formula I, formula II, formula III, formula IV or formula V or the pharmaceutically acceptable salt thereof, R_{5b} is selected from the group consisting of halogen, C₁₋₆ alkyl, C₃₋₆ cycloalkyl, hydroxy, cyano, amino and nitro; preferably, R_{5b} is selected from the group consisting of halogen, methyl, ethyl, n-propyl, isopropyl, cyclopropyl, hydroxy, cyano, amino and nitro; and more preferably, R_{5b} is selected from the group consisting of fluorine, methyl, hydroxy, cyano, amino and nitro.

In a second aspect, the present disclosure also provides a compound of the following formula or a pharmaceutically acceptable salt thereof,

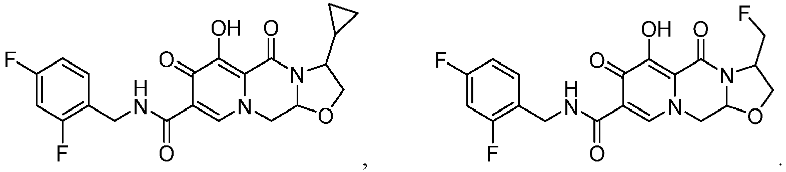

In a third aspect, the present disclosure also provides a compound of the following formula or a pharmaceutically acceptable salt thereof,

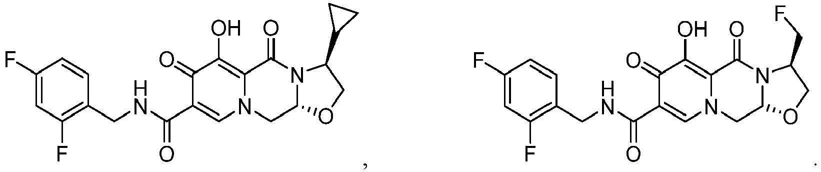

In a fourth aspect, the present disclosure also provides an isotope substitute of the compound of formula I, formula II, formula III, formula IV, formula V, the second aspect or the third aspect or the pharmaceutically acceptable salt thereof; preferably, the isotope substitute is deuterium atom substitute.

In a fifth aspect, the present disclosure also provides a pharmaceutical composition comprising the compound of formula I, formula II, formula III, formula IV, formula V, the compound in the second aspect, the third aspect, the fourth aspect or the pharmaceutically acceptable salt thereof, and pharmaceutically acceptable excipient(s).

In some embodiments, the unit dose of the pharmaceutical composition is 0.001 mg to 1000 mg.

In some embodiments, the pharmaceutical composition comprises 0.01 to 99.99% of the aforementioned compound or the pharmaceutically acceptable salt thereof relative to the total weight of the composition. In some embodiments, the pharmaceutical composition comprises 0.1 to 99.9% of the aforementioned compound or the pharmaceutically acceptable salt thereof. In some embodiments, the pharmaceutical composition comprises 0.5% to 99.5% of the compound or the pharmaceutically acceptable salt thereof. In some embodiments, the pharmaceutical composition comprises 1% to 99% of the compound or the pharmaceutically acceptable salt thereof. In some embodiments, the pharmaceutical composition comprises 2% to 98% of the compound or the pharmaceutically acceptable salt thereof. The present disclosure also provides a method for treating or preventing HIV infection in a human, comprising a step of administering to the human a therapeutically effective amount of the compound of formula I, formula II, formula III, formula IV, formula V, the compound in the second aspect, the third aspect, the fourth aspect or the pharmaceutically acceptable salt thereof or the pharmaceutical composition in the fifth aspect.

The present disclosure also provides the compound of formula I, formula II, formula III, formula IV, formula V, the compound in the second aspect, the third aspect, the fourth aspect or the pharmaceutically acceptable salt thereof or the pharmaceutical composition in the fifth aspect for use in treating or preventing HIV infection in a human, the compound or the pharmaceutically acceptable salt thereof or the pharmaceutical composition is administered in combination with one or more additional therapeutic agent(s).

In some embodiments, the additional therapeutic agent is one or more selected from the group consisting of HIV protease inhibitor, non-nucleoside inhibitor of HIV reverse transcriptase, nucleoside inhibitor of HIV reverse transcriptase and nucleotide inhibitor of HIV reverse transcriptase.

In some embodiments, the additional therapeutic agent is one or more selected from the group consisting of raltegravir, lamivudine, abacavir, ritonavir, dolutegravir, darunavir, atazanavir, emtricitabine, tenofovir, elvitegravir, rilpivirine and lopinavir.

Although all of the above formulas are drawn as certain isomeric forms for simplicity, the present invention may include all isomers, such as tautomers, rotamers, geometric isomers, diastereomers, racemates and enantiomers.

Tautomers are structural isomers of organic compounds that readily interconvert by a chemical reaction called tautomerization. This reaction often results in the formal migration of hydrogen atoms or protons accompanied by the conversion between a single bond and an adjacent double bond. Some common tautomeric pairs include: keto-enol and lactam-lactim. An example of a lactam-lactim equilibrium is present between A and B as shown below.

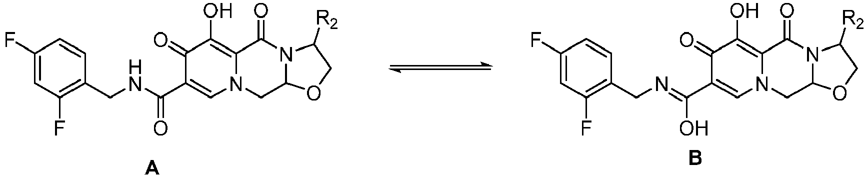

All compounds in the present invention can be drawn as form A or form B. All tautomeric forms are within the scope of the present invention. The nomenclature of the compounds does not exclude any tautomers.

"Optionally" or "optional" means that the event or circumstance described subsequently can, but need not, occur, and such a description includes the situation in which the event or circumstance does or does not occur. For example, "the C₁-C₆ alkyl optionally substituted by halogen or cyano" means that halogen or cyano can be, but need not be, present, and such a description includes the situation of the alkyl being substituted by halogen or cyano and the alkyl being not substituted by halogen and cyano.

In the chemical structure of the compound described in the present invention, a " 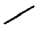 " bond is not specified with a configuration, that is, if chiral isomers exist in the chemical structure, a " 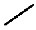 " bond may be " 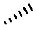 " or " 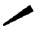 ", or includes both " 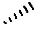 " and " 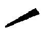 " configurations. In the chemical structure of the compound described in the present disclosure, a " 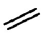 " bond is not specified with a configuration, that is it may be in a Z configuration or an E configuration, or includes both configurations.

Any isotopically-labeled derivative of the compound or the pharmaceutically acceptable salt or isomer thereof described in the present disclosure is encompassed by the present disclosure. Atoms that can be isotopically labeled include, but are not limited to, hydrogen, carbon, nitrogen, oxygen, phosphorus, fluorine, chlorine, iodine, etc. They may be separately replaced by the isotopes ²H (D), ³H , ¹¹C, ¹³C, ¹⁴C, ¹⁵N, ¹⁸F , ³¹P, ³²P, ³⁵S, ³⁶Cl, ¹²⁵I, etc. Unless otherwise stated, when a position is specifically designated as deuterium (D), that position shall be understood to be deuterium having an abundance that is at least 3000 times greater than the natural abundance of deuterium (which is 0.015%) (i.e., incorporating at least 45% deuterium).

### Term definitions:

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

The term "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration for acceptable use in humans or livestock.

The term "alkyl" refers to a saturated aliphatic hydrocarbon group, which is a straight or branched chain group comprising 1 to 20 carbon atoms. An alkyl having 1 to 6 carbon atoms is preferred. Non-limiting examples include methyl, ethyl, n-propyl, isopropyl, n-butyl, isobutyl, *tert*-butyl, *sec*-butyl, *n*-pentyl, 1,1-dimethylpropyl, 1,2-dimethylpropyl, 2,2-dimethylpropyl, and various branched isomers thereof. Unless otherwise stated, the alkyl can be substituted or unsubstituted. When substituted, the substituent(s) can be substituted at any available connection point. The substituent(s) is preferably one or more groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkyl, 3 to 6 membered heterocyclyl, C₅₋₈ cycloalkenyl, C₃₋₆ cycloalkoxy, 3 to 6 membered heterocycloalkoxy, C₅₋₈ cycloalkenyloxy, C₆₋₁₀ aryl and 5 to 6 membered heteroaryl, the C₁₋₆ alkyl, C₁₋₆ alkoxy, C₂₋₆ alkenyloxy, C₂₋₆ alkynyloxy, C₃₋₆ cycloalkyl, 3 to 6 membered heterocyclyl, C₅₋₈ cycloalkenyl, C₃₋₆ cycloalkoxy, 3 to 6 membered heterocycloalkoxy, C₅₋₈ cycloalkenyloxy, C₆₋₁₀ aryl and 5 to 6 membered heteroaryl are each optionally substituted by one or more substituents selected from the group consisting of halogen, deuterium, hydroxy, oxo, nitro and cyano.

The term "cycloalkyl" or "cyclic hydrocarbon" refers to a saturated or partially unsaturated monocyclic or polycyclic hydrocarbon substituent having 3 to 20 carbon atoms, and preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl and the like. Polycyclic cycloalkyl includes a cycloalkyl having a spiro ring, fused ring or bridged ring.

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. Unless otherwise stated, the cycloalkyl can be optionally substituted or unsubstituted. When substituted, the substituent(s) is preferably one or more groups independently selected from the group consisting of deuterium, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" or "heterocycle" refers to a saturated or partially unsaturated monocyclic or polycyclic hydrocarbon group having 3 to 20 ring atoms, wherein one or more ring atoms are heteroatoms selected from the group consisting of N, O and S(O)ₘ (wherein m is an integer from 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 7 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like. Polycyclic heterocyclyl includes a heterocyclyl having a spiro ring, fused ring or bridged ring. Non-limiting examples of "heterocyclyl" include: 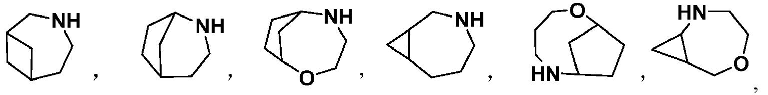 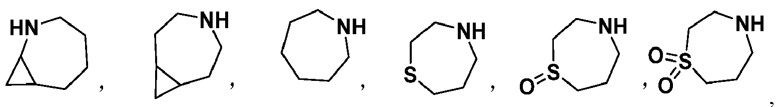 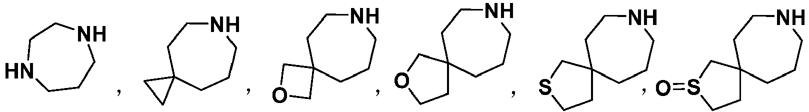 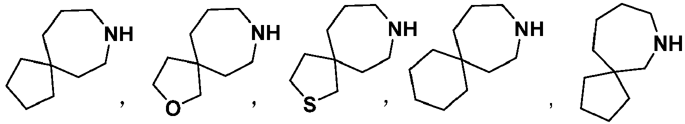 and the like.

The heterocyclyl can be optionally substituted or unsubstituted. When substituted, the substituent(s) is preferably one or more groups independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkyl, 3 to 6 membered heterocyclyl, C₅₋₈ cycloalkenyl, C₃₋₆ cycloalkoxy, 3 to 6 membered heterocycloalkoxy, C₅₋₈ cycloalkenyloxy, C₆₋₁₀ aryl and 5 to 6 membered heteroaryl, the C₁₋₆ alkyl, C₁₋₆ alkoxy, C₂₋₆ alkenyloxy, C₂₋₆ alkynyloxy, C₃₋₆ cycloalkyl, 3 to 6 membered heterocyclyl, C₅₋₈ cycloalkenyl, C₃₋₆ cycloalkoxy, 3 to 6 membered heterocycloalkoxy, C₅₋₈ cycloalkenyloxy, C₆₋₁₀ aryl and 5 to 6 membered heteroaryl are each optionally substituted by one or more substituents selected from the group consisting of halogen, deuterium, hydroxy, oxo, nitro and cyano.

The heterocyclyl ring can be fused to the ring of aryl, heteroaryl or cycloalkyl, wherein the ring bound to the parent structure is heterocyclyl, and non-limiting examples thereof include: 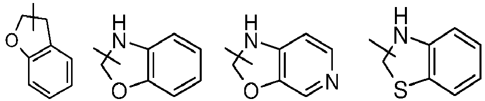 and the like.

The expression "substituted by one or more A, B......" refers to being substituted by one or more substituent(s). When substituted by multiple substituents, it can refer to a plurality of the same substituents, or a combination of one or a plurality of different substituents.

### DESCRIPTION OF THE DRAWINGS

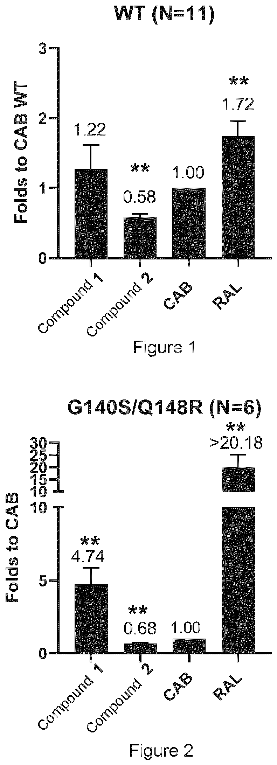

Figure 1: Antiviral activity EC50 of compound 1 and compound 2 against WT (wild type) strain.
Figure 2: Antiviral activity EC50 of compound 1 and compound 2 against G140S/Q148R strain.

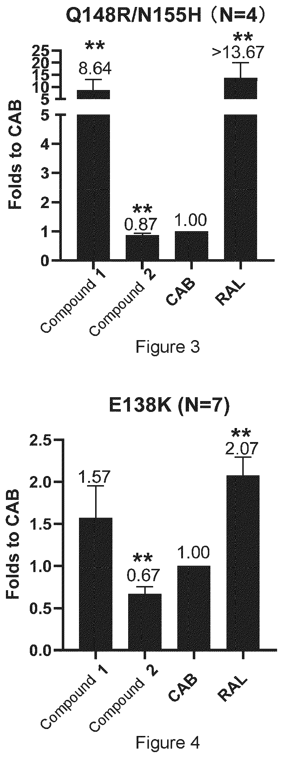

Figure 3: Antiviral activity EC50 of compound 1 and compound 2 against Q148R/N155H strain.
Figure 4: Antiviral activity EC50 of compound 1 and compound 2 against E138K strain.

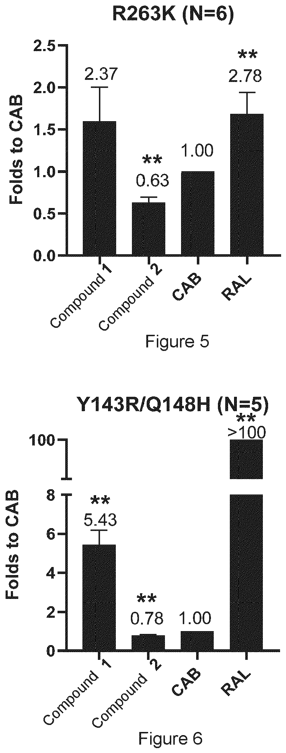

Figure 5: Antiviral activity EC50 of compound 1 and compound 2 against R263K strain.
Figure 6: Antiviral activity EC50 of compound 1 and compound 2 against Y143R/Q148H strain.

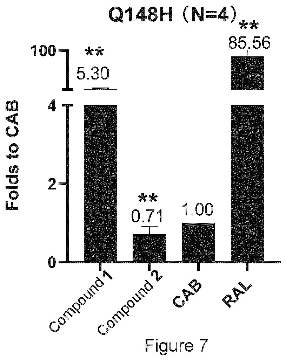

Figure 7: Antiviral activity EC50 of compound 1 and compound 2 against Q148H strain.

In the figures, RAL represents raltegravir; CAB represents cabotegravir; the antiviral activity EC50 of CAB against each mutant virus strain was set to 1; **: significantly different from CAB and p-value is less than 0.05; N represents the number of repeated detections.

### DETAILED DESCRIPTION OF THE INVENTION

The present disclosure will be further described with reference to the following examples, but the examples should not be considered as limiting the scope of the present disclosure.

Experimental procedures without conditions specified in the examples of the present disclosure are generally conducted according to conventional conditions, or according to conditions recommended by the manufacturers of the starting materials or commercial products. Reagents without specific origins indicated are commercially available conventional reagents.

The structures of the compounds were identified by nuclear magnetic resonance (NMR) and/or mass spectrometry (MS). NMR shifts (δ) were given in 10⁻⁶ (ppm). NMR was determined by a Bruker AVANCE-400 machine. The solvents for determination are deuterated-dimethyl sulfoxide (DMSO-*d*₆), deuterated-chloroform (CDCl₃) and deuterated-methanol (CD₃OD), and the internal standard is tetramethylsilane (TMS). The spatial configurations of the optical isomers (isomers) of the compounds can be further confirmed by determining single crystal parameters.

HPLC was determined on a Waters ACQUITY ultra high performance LC, Shimadzu LC-20A systems, Shimadzu LC-2010HT series, or Agilent 1200 LC high performance liquid chromatograph (ACQUITY UPLC BEH C18 1.7UM 2.1×50MM column, Ultimate XB-C18 3.0*150 mm column, or Xtimate C18 2.1*30 mm column).

MS was determined on Waters SQD2 mass spectrometer in positive/negative ion mode with a mass scan range of 100 to 1200.

Chiral HPLC was determined on a Chiralpak IC-3 100×4.6 mm I.D., 3 µm, Chiralpak AD-3 150×4.6 mm I.D., 3 µm, Chiralpak AD-3 50×4.6 mm I.D., 3 µm, Chiralpak AS-3 150×4.6 mm I.D., 3 µm, Chiralpak AS-3 100×4.6 mm I.D., 3 µm, ChiralCel OD-3 150×4.6 mm I.D., 3 µm, Chiralcel OD-3 100×4.6 mm I.D., 3 µm, ChiralCel OJ-H 150×4.6 mm I.D., 5 µm, ChiralCel OJ-3 150×4.6 mm I.D., 3 µm chromatographic column.

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification of thin-layer chromatography separation was 0.4 mm to 0.5 mm.

The flash column purification was performed using a Combiflash Rf150 (TELEDYNE ISCO) or Isolara one (Biotage) system.

Normal phase column chromatography was generally performed using 100-200 mesh, 200-300 mesh or 300-400 mesh Yantai Huanghai silica gel as a carrier, or using a Changzhou Santai pre-filled ultrapure normal phase silica gel column (40-63 µm, 60 g, 12 g, 25 g, 40 g, 80 g or other specifications).

Reverse phase column chromatography was generally performed using a Changzhou Santai pre-filled ultrapure C18 silica gel column (20-45 µm, 100 Å, 40 g, 80 g, 120 g, 220 g or other specifications).

The high pressure column purification was performed using a Waters AutoP system equipped with Waters XBridge BEH C18 OBD Prep Column, 130 Å, 5 µm, 19 mm×150 mm or Atlantis T3 OBD Prep Column, 100 Å, 5 µm, 19 mm×150 mm.

The chiral preparation was performed using a DAICEL CHIRALPAK IC (250 mm*30 mm, 10 µm) or Phenomenex-Amylose-1 (250 mm*30 mm, 5 µm) column.

The known starting materials of the present disclosure can be prepared by the known methods in the art, or can be purchased from Shanghai Titan Scientific, ABCR GmbH & Co. KG, Acros Organics, Aldrich Chemical Company, Accela ChemBio Inc., Dari Chemical Company etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reaction was performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). Regarding the developing solvent used in the reactions, the eluent system in column chromatography and the developing solvent system in thin layer chromatography for purification of the compounds, the ratio of the volume of the solvents was adjusted according to the polarity of the compounds, and a small quantity of alkaline reagent such as triethylamine or acidic reagent such as acetic acid could also be added for adjustment.

### Example 1

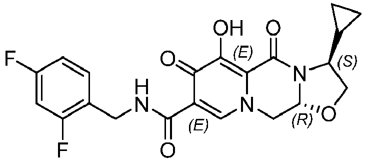

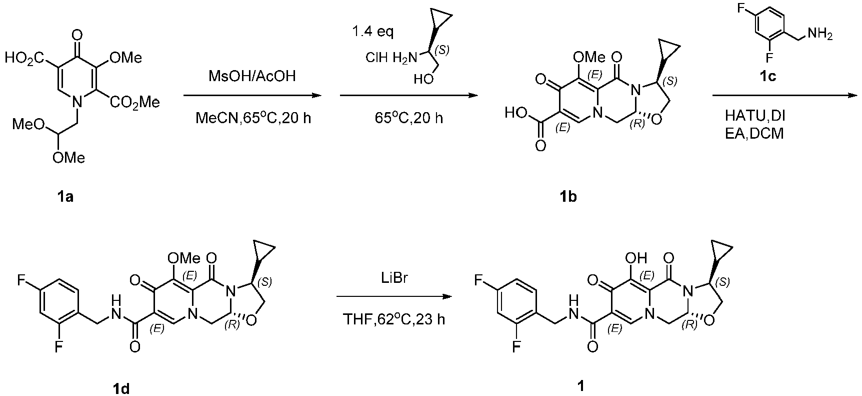

### 1. Synthesis of compound 1b

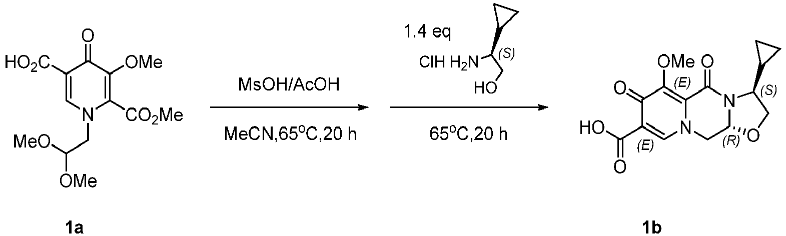

**1a** (250.4 mg, 0.794 mmol, 1.0 eq) was added to a 10 mL reaction flask under a nitrogen atmosphere, followed by the addition of 2.5 mL of acetonitrile, AcOH (227 µL) and MsOH (16 µL). After completion of the addition, the reaction solution was stirred in a oil bath at 66°C for about 20 hours. A sample was taken for LCMS detection. (S)-2-Amino-2-cyclopropylethanol hydrochloride (153.3 mg, 1.114 mmol, 1.4 eq) was added, and after about 5 minutes, potassium carbonate (158.3 mg, 1.145 mmol, 1.4 eq) and MeCN (2.1 mL) were added. The reaction solution was stirred in a oil bath at 66°C for another 20 hours. A sample was taken for LCMS detection. The reaction was completed and stopped. The reaction solution was cooled and concentrated to dryness under reduced pressure. The residue was diluted with 1 N HCl and DCM, and separated into two phases. The aqueous phase was extracted with DCM, and the organic phases were combined, dried over anhydrous sodium sulfate, filtered and concentrated to obtain the crude product **1b** (240 mg), which was directly used in the next step. Ms (ESI): m/z 321.1 [M+1]⁺.

### 2. Synthesis of compound 1d

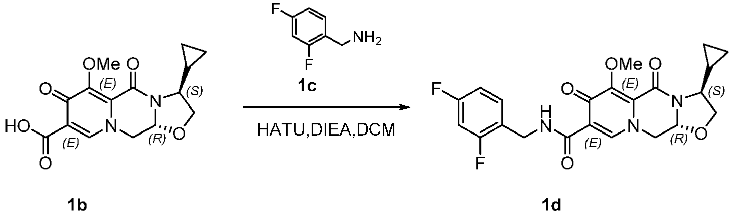

**1b** (300 mg, 0.94 mmol, 1.0 eq) and **1c** (158.4 mg, 1.1 mmol, 1.2 eq) were added to a 25 mL eggplant-shaped flask at room temperature, followed by the addition of 5 mL of DCM. DIEA (302 mg, 2.34 mmol, 2.5 eq) and HATU (448.3 mg, 1.18 mmol, 1.3 eq) were added under stirring, and then the reaction solution was stirred under a nitrogen atmosphere at room temperature for 2 to 3 hours. A sample was taken for LCMS detection. The reaction was completed and stopped. The reaction solution was directly mixed with silica gel, and purified by column chromatography to obtain the crude oily product **1d** (786 mg) with a yield of 188%, which was directly used in the next step. Ms (ESI): m/z 446.1 [M+1]⁺.

### 3. Synthesis of compound 1

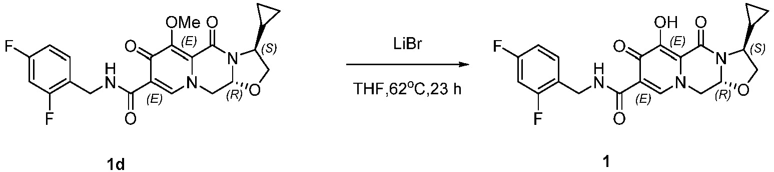

The crude product **1d** (739.2 mg, 1.0 eq), LiBr (368 mg, 4.24 mmol, 2.5 eq) and THF (10 mL) were added to a 50 mL eggplant-shaped flask at room temperature, and stirred in a oil bath at 62 to 65°C for about 24 hours. A sample was taken for LCMS detection. The reaction was completed and stopped. The reaction solution was cooled and concentrated to remove THF. 3 N HCl (3 mL) was added to the residue in an ice bath, and the resulting mixture was diluted with DCM (3 mL), separated into two phases, and the aqueous phase was extracted with DCM. The organic phases were combined, washed once with saturated aqueous sodium bicarbonate solution (1.5 mL), dried over anhydrous sodium sulfate and filtered. The filtrate was concentrated to dryness under reduced pressure, purified by preparative HPLC, and lyophilized to obtain compound 1 (270 mg). Ms (ESI): m/z 432.1 [M+1]⁺. ¹H-NMR (400 MHz, CDCl₃) δ 10.32-10.29 (t, 1H), 8.35 (s, 1H), 7.39-7.33 (q, 1H), 6.84-6.77(m, 2H), 5.44-5.41 (m, 1H), 4.69-4.59 (m, 2H), 4.44-4.40 (q, 1H), 4.32-4.28 (q, 1H), 4.02-3.98 (q, 1H), 3.92-3.86 (m, 2H), 1.07-1.03 (m, 1H), 0.79-0.62 (m, 3H), 0.37-0.30 (m, 1H).

### Example 2

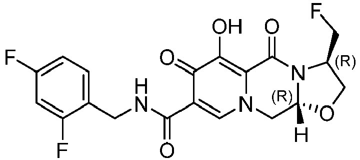

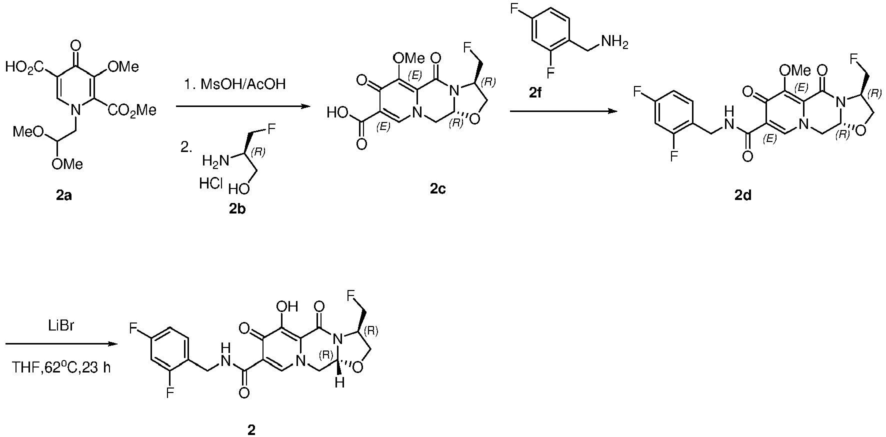

### 1. Synthesis of compound 2b

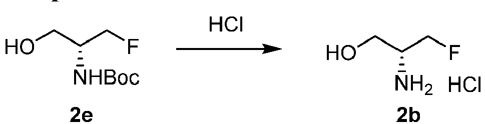

**2e** (503 mg, 2.6 mmol) (prepared according to WO2020167628) and HCl/dioxane (4 M, 2.6 ml, 10.4 mmol) were added to a 25 mL eggplant-shaped flask at room temperature (20°C), and stirred at room temperature (20°C) for 16 hours. The reaction was completed according to LCMS monitoring. The reaction solution was concentrated to obtain compound **2b** (320 mg) with a yield of 95%, which was directly used in the next step.

### 2. Synthesis of compound 2c

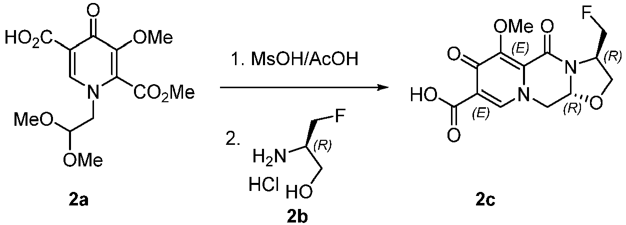

**2a** (399 mg, 1.266 mmol, 1.0 eq) was added to a 25 mL reaction flask under a nitrogen atmosphere, followed by the addition of 4 mL of acetonitrile, AcOH (364 µL) and MsOH (28.8 µL). After completion of the addition, the reaction solution was stirred in a oil bath at 66°C for about 24 hours. A sample was taken for LCMS detection, showing that the remaining starting materials were less than 1%. 2c (234 mg, 1.806 mmol, 1.4 eq) was added, and after about 10 minutes, potassium carbonate (284.6 mg, 2.059 mmol, 1.6 eq) was added. The reaction solution was stirred in a oil bath at 66°C for another 21 hours. A sample was taken for LCMS detection. The reaction was completed and stopped. The reaction solution was cooled and concentrated to dryness under reduced pressure. The residue was diluted with 1 N HCl and DCM, separated into two phases, and the aqueous phase was extracted with DCM. The organic phases were combined, dried over anhydrous sodium sulfate, filtered and concentrated to obtain the crude product **2c** (420 mg), which was directly used in the next step. Ms (ESI): m/z 313.0 [M+1]⁺.

### 3. Synthesis of compound 2d

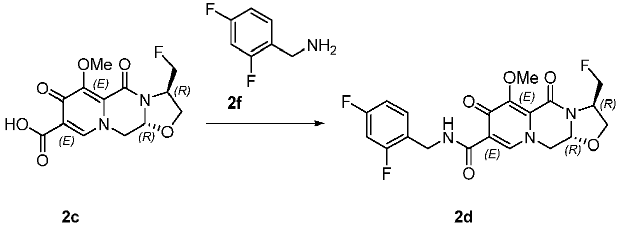

**2c** (450 mg, 1.44 mmol, 1.0 eq) and **2f** (222.6 mg, 1.56 mmol, 1.1 eq) were added to a 50 mL eggplant-shaped flask at room temperature, followed by the addition of 8 mL of DCM. DIEA (385 mg, 2.98 mmol, 2.1 eq) and HATU (641.5 mg, 1.68 mmol, 1.2 eq) were added under stirring, and then the reaction solution was stirred under a nitrogen atmosphere at room temperature for 2 to 3 hours. A sample was taken for LCMS detection. The reaction was completed and stopped. The reaction solution was directly mixed with silica gel, and purified by column chromatography to obtain the crude oily product **2d** (877 mg) with a yield of 139%, which was directly used in the next step. Ms (ESI): m/z 438.1 [M+1]⁺.

### 4. Synthesis of compound 2

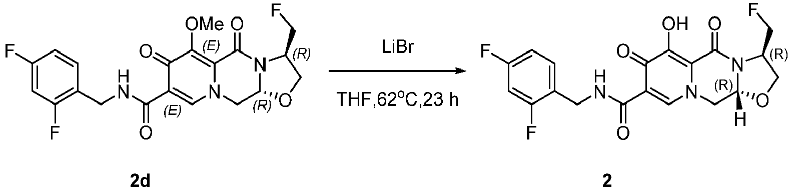

The crude product **2d** (754 mg), LiBr (386.3 mg, 4.448 mmol) and THF (10 mL) were added to a 50 mL eggplant-shaped flask at room temperature, and stirred in a oil bath at 62 to 65°C for about 24 hours. A sample was taken for LCMS detection. The reaction was completed and stopped. The reaction solution was cooled and concentrated to remove THF. 3 N HCl (3 mL) was added to the residue in an ice bath, and the resulting mixture was diluted with DCM (3 mL), separated into two phases, and the aqueous phase was extracted with DCM. The organic phases were combined, washed once with saturated aqueous sodium bicarbonate solution (1.5 mL), dried over anhydrous sodium sulfate and filtered. The filtrate was concentrated to dryness under reduced pressure to obtain 506 mg of the crude product, which was purified by preparative HPLC and lyophilized to obtain compound 2 (44 mg). Ms (ESI): m/z 424.1 [M+1]⁺. ¹H-NMR (400 MHz, CDCl₃) δ10.26-10.20 (m, 1H), 8.35 (s, 1H), 7.39-7.31 (m, 1H), 6.84-6.78(m, 2H), 5.33-5.30 (m, 1H), 5.10-5.07 (q, 0.5H), 4.98-4.95 (q, 0.5H), 4.65-4.41 (m, 6H), 4.25-4.21 (m, 1H), 3.97-3.91 (m, 1H).

### Test Example 1:

The protective effect of the test compound on MT-4 cells infected by human immunodeficiency virus type 1 IIIB strain (HIV-1IIIB) at different concentrations was evaluated.

### Materials and methods

The test compound was dissolved in DMSO at a concentration of 10 mM and stored at -20°C.
1) Test of cytotoxicity of the compound: The test compound was evaluated using a starting concentration of 100 nM and five serial half-log dilutions. Each compound was present in the test medium at a concentration 2 folds of the final desired test concentration. The compound was diluted to 1 mM in DMSO. The compound was then diluted to 200 nM in a drug dilution tube containing 998 µL of test medium (adding 2 µL of 1 mM stock). 320 microliters (320 µL) of the 200 nM solution was transferred into 680 µL of the test medium (half-log dilution), and a total of five serial dilutions were carried out. 100 microliters (100 µL) of the solution of each concentration was added to duplicate wells for cytotoxicity evaluation, and each concentration was added to one well for colorimetric evaluation.
2) Test of the anti-HIV-1 IIIB infection activity of the compound: A starting concentration of 10 nM was used, and five serial five-fold dilutions were carried out on the compound. Each compound was present in the test medium at a concentration 2 folds of the final desired test concentration. 100 microliters (100 µL) of the solution of each concentration was added to triplicate wells for efficacy evaluation, and each concentration was added one well for colorimetric evaluation. AZT was purchased from Sigma Aldrich (St. Louis, MO, USA), and evaluated as a control compound in the antiviral assay.

### Cell protection assay against HIV infection

Cell culture: MT-4 cells were passaged in a T-75 flask before use in the antiviral assay. On the day before the assay, cells were passaged in aliquots at a 1:2 ratio to ensure that they were in the exponential growth phase at the time of infection. Total cells and viable cells were quantified using a hemocytometer and trypan blue dye. The proportion of viable cells used for the assay was greater than 95%. The cells were resuspended in tissue culture medium at 5 × 10⁴ cells per milliliter, and the resulting suspension was added to a drug-containing microtiter plate in a volume of 50 µL.

Virus preparation: The virus used for this assay was lymphotropic virus strain HIV-1IIIB. The viruses were amplified in CEM-SS cells for the preparation of virus stock solution. The pre-titered virus aliquot tube was taken from the refrigerator (-80°C), and slowly thawed to room temperature in a biosafety cabinet. The viruses were diluted in the tissue culture medium. 50 µl of the diluted virus would produce 85% to 95% cell killing 6 days after infection. 50 µL of the diluted virus was added immediately to the microtiter plate containing cells and drugs.

Evaluation of the efficacy and toxicity of the compound with XTT: After culturing for six days in a 37°C, 5% CO₂ incubator, tetrazolium dye XTT (2,3-bis(2-methoxy-4-nitro-5-sulfophenyl)-5-[(phenylamino)carbonyl]-2H-tetrazolium hydroxide) was added to the cell culture plate to conduct the test. The XTT solution was a 1 mg/ml RPMI1640 stock solution prepared on the same day. Phenazine methyl sulfate (PMS) solution at a concentration of 0.15 mg/ml was prepared in PBS, and stored in the dark at -20°C. XTT/PMS stock solution was prepared before use by adding 40 µl of PMS per milliliter of XTT solution. 50 µl of XTT/PMS was added to each well of the plate, and the plate was re-incubated for 4 hours at 37°C. The plate was sealed with adhesive plate sealant, and gently shaked or inverted several times to mix the soluble formazan product. The absorbance was read at 450/650 nm.

**Table 1 Antiviral efficacy and cytotoxicity data.**

| | **EC₅₀ (nM)** | **CC₅₀ (nM)** | **CC₅₀/EC₅₀** |
|---|---|---|---|
| AZT (Control compound) | 2 | >500 | >250 |
| Cabotegravir | 2.40 | >100 | >41.7 |
| Compound 1 | 3.12 | >100 | >32.1 |
| Compound 2 | 0.89 | >100 | >112 |

### Test Example 2:

The inhibitory activity against pseudoviruses containing common clinical mutation sites of HIV-1 integrase (G140S/Q148R, Q148R/N155H, E138K, Q148H, R263K and Y143R/Q148H) was determined.

### Pseudovirus-based assay of anti-HIV-1 activity of compounds

Preparation of pseudovirus: Using the lentiviral plasmid pNL4-3-Luc-R⁻E⁻ (HedgehogBio) as the backbone, an integrase point mutation plasmid was constructed. HEK293T cells (ATCC) were seeded in a 6-well plate at 5×10⁵ cells per well. After 24 hours, when the cell density reached 60% to 80%, transfection was performed. pNL4-3-Luc-R⁻E⁻ or pNL4-3-Luc-R⁻E⁻-IN-mutant plasmid and lentivirus packaging plasmid pMD2.G (Beijing Zoman Bio) were added to the transfection reagent in a certain proportion. Cell transfection was performed according to the instructions of transfection reagent PEI25K (Maokang Biotechnology). After 48 hours, the supernatant was collected, centrifuged at 3000 rpm for 5 minutes, filtered with a 0.45 µm microporous membrane, aliquoted, and stored at -80°C for later use. In HEK293T cells, the pseudovirus titer was determined based on luciferase activity.

Determination of antiviral activity of compounds: In a 96-well V-bottom plate, the compound to be tested was diluted with DMSO semi-logarithmically, and a compound solution 4 times the final concentration was prepared for later use. According to the results of different virus titrations, the virus was diluted with complete culture medium for later use. HEK293T cells were digested with trypsin to obtain a cell suspension of 2 ×10⁵/mL. 50 µl of compound diluent, 50 µl of virus diluent and 100 µl of cell suspension were added successively to a 96-well flat-bottomed plate. The final concentration of the compound was 1000 nM to 0.1 nM. The 96-well plate was incubated in a 37°C, 5% CO₂ incubator. After 72 hours, 100 µL of culture supernatant was pipetted out carefully from each well of the 96-well plate, 25 µL of Steady-Glo detection reagent (Promega, Cat#E2550) was added to each well in the dark, and then assayed on a machine (Tecan, Type#Spark).

**Table 2 EC₅₀ and EC₉₀ data of compound 1, compound 2 and cabotegravir against different mutant strains**

| | Compound 1 | | Compound 2 | | Cabotegravir | | Raltegravir | |
|---|---|---|---|---|---|---|---|---|
| | EC₅₀(nM) | EC₉₀(nM) | EC₅₀(nM) | EC₉₀(nM) | EC₅₀(nM) | EC₉₀(nM) | EC₅₀(nM) | EC₉₀(nM) |
| WT (wild type) | 3.3 | 29.1 | 1.8 | 12.9 | 3.0 | 21.8 | 5.5 | 60.7 |
| E138K | 5.1 | 27.1 | 2.3 | 14.1 | 3.7 | 24.4 | 8.1 | 46.2 |
| Q148H | 15.9 | 105.3 | 2.2 | 12.5 | 3.6 | 15.4 | 214.2 | >1000 |
| R263K | 6.5 | 58.3 | 2.9 | 25.1 | 4.9 | 58.8 | 7.1 | 68.0 |
| Y143R/Q148H | 21.3 | 193.1 | 3.1 | 14.1 | 4.2 | 24.8 | >1000 | >1000 |
| G140S/Q148R | 243.8 | >1000 | 42.2 | 193.8 | 63.5 | 301.0 | >1000 | >1000 |
| Q148R/N155H | 610.7 | >1000 | 82.3 | >1000 | 98.0 | >1000 | >1000 | >1000 |

Against common clinical integrase mutant strains (G140S/Q148R, Q148R/N155H, E138K, Q148H, R263K and Y143R/Q148H), the activity of compound 2 is better than that of cabotegravir with significant differences.

### Test Example 3: Inhibitory activity assay against clinical HIV-1 strains.

Preparation of virus: Clinical HIV-1 virus strains (Table 3) were obtained from the US NIH AIDS Research and Reference Reagent Library. PBMC were extracted from three fresh blood samples that were HIV and HBV seronegative, mixed and cultured, and PHA-P (phytohemagglutinin) was added to stimulate activation. The cells and virus were incubated at 37°C/5% CO₂, and the cell culture supernatant after infection was collected and stored frozen at -80°C. Each viral titer was titrated in pooled PBMCs from three donors.

### Assay of antiviral activity of compounds:

| **HIV-1 subtypes** | **Virus strains** |
|---|---|
| A | RW/92/009 |
| C | MW/93/959 |
| D | UG/92/001 |

Assay of antiviral activity of compounds: Frozen PBMCs were resuspended in fresh culture medium (1 × 10⁶ cells/mL), and added to a round-bottom 96-well plate (50 µL/well). 100 µl of culture medium containing 2 times the final concentration of the compound was added to each well, and then 50 µL of virus solution was added (the final infection dose was MOI=0.002). After 7 days of culture, the reverse transcriptase activity in the supernatant was determined to evaluate HIV-1 replication. At the same time, a 96-well culture plate with only compounds and cells was prepared for the assay of compound cytotoxicity.

In human primary PBMC, the CC₅₀ of the compounds were all greater than 100 nM. Against different subtypes of HIV-1 clinical isolates (RW/92/009, MW/93/959, UG/92/001), compound 2 was more active than cabotegravir in human PBMC.

**Table 3: Antiviral activity data**

| Virus strains | hPBMC/HIV-1 EC₅₀ (nM) | | | hPBMC/HIV-1 EC₉₀ (nM) | | |
|---|---|---|---|---|---|---|
| | AZT | Cabotegravir | Compound 2 | AZT | Cabotegravir | Compound 2 |
| RW/92/009 | 3.58 | 0.09 | 0.02 | 75.5 | 0.59 | 0.40 |
| MW/93/959 | 0.31 | 0.06 | 0.007 | 38.6 | 0.67 | 0.37 |
| UG/92/001 | 4.02 | 0.46 | 0.28 | 62.0 | 2.94 | 1.28 |

## Claims

1. A compound of formula I or a pharmaceutically acceptable salt thereof 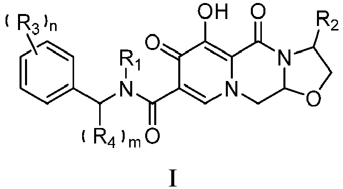 wherein,
R₁ is selected from the group consisting of hydrogen, C₁₋₆ alkyl, C₃₋₆ cycloalkyl and hydroxy, the C₁₋₆ alkyl and C₃₋₆ cycloalkyl are each optionally substituted by one to three R₅ₐ;
R₅ₐ is selected from the group consisting of halogen, C₁₋₆ alkyl, C₃₋₆ cycloalkyl, hydroxy, cyano, amino and nitro;
R₂ is selected from the group consisting of hydrogen, halogen, C₁₋₆ alkyl substituted by 1 to 3 halogen, C₃₋₆ cycloalkyl, 3 to 6 membered heterocyclyl, hydroxy, methylenecyclopropyl and oxo, the C₃₋₆ cycloalkyl, 3 to 6 membered heterocyclyl and methylenecyclopropyl are each optionally substituted by one to three R_{5b};
R_{5b} is selected from the group consisting of halogen, C₁₋₆ alkyl, C₃₋₆ cycloalkyl, hydroxy, cyano, amino and nitro;
each R₃ is independently selected from the group consisting of halogen, cyano, amino, nitro, C₁₋₆ alkyl, C₃₋₆ cycloalkyl and hydroxy, the C₁₋₆ alkyl and C₃₋₆ cycloalkyl are each optionally substituted by one to three R_{5c};
n is an integer selected from 0 to 4;
each R_{5c} is independently selected from the group consisting of halogen, C₁₋₆ alkyl, C₃₋₆ cycloalkyl, hydroxy, cyano, amino and nitro;
each R₄ is independently selected from the group consisting of halogen, cyano, amino, nitro, C₁₋₆ alkyl, C₃₋₆ cycloalkyl, hydroxy and oxo;
m is an integer from 0 to 2; preferably, m is an integer from 0 to 1; and more preferably, m is 0.

2. The compound or the pharmaceutically acceptable salt thereof according to claim 1, wherein
each R₃ is independently selected from the group consisting of halogen, cyano, amino, nitro, methyl, ethyl, cyclopropyl, isopropyl and hydroxy, the methyl, ethyl, cyclopropyl and isopropyl are each optionally substituted by one to three R_{5c};
n is an integer selected from 0 to 2;
each R_{5c} is independently selected from the group consisting of halogen, methyl, ethyl, cyclopropyl, isopropyl, hydroxy, cyano, amino and nitro;
most preferably, each R₃ is independently selected from halogen; n is an integer selected from 0 to 2.

3. The compound or the pharmaceutically acceptable salt thereof according to claim 1, wherein the compound of formula I is selected from 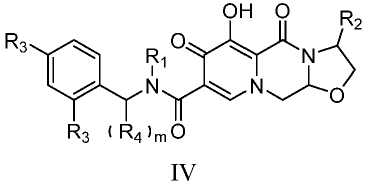 wherein
R₁, R₂, R₃, R₄ and m are as defined in claim 1.

4. The compound or the pharmaceutically acceptable salt thereof according to claim 1, wherein the compound of formula I is selected from 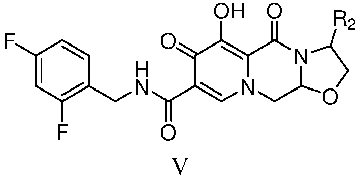 R₂ is as defined in claim 1.

5. The compound or the pharmaceutically acceptable salt thereof according to any one of claims 1 to 4, wherein
R₂ is selected from the group consisting of hydrogen, halogen, C₁₋₆ alkyl substituted by 1 to 3 halogen, C₃₋₆ cycloalkyl, hydroxy, methylenecyclopropyl and oxo, the C₃₋₆ cycloalkyl and methylenecyclopropyl are each optionally substituted by one to three R_{5b}, R_{5b} is as defined in claim 1;
preferably, R₂ is selected from the group consisting of hydrogen, halogen, C₁₋₆ alkyl substituted by 1 to 3 halogen, and C₃₋₆ cycloalkyl, the C₃₋₆ cycloalkyl is optionally substituted by one to three R_{5b}, R_{5b} is as defined in claim 1;
more preferably, R₂ is selected from the group consisting of methyl substituted by 1 to 3 fluorine, and cyclopropyl, the cyclopropyl is optionally substituted by one to three halogen;
most preferably, R₂ is selected from the group consisting of monofluoromethyl and cyclopropyl.

6. The compound or the pharmaceutically acceptable salt thereof according to claim 1, wherein the compound of formula I is selected from the group consisting of

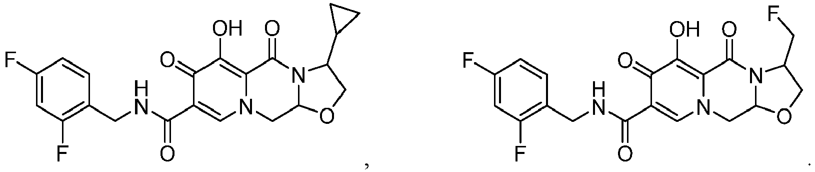

7. The compound or the pharmaceutically acceptable salt thereof according to claim 1, wherein the compound of formula I is selected from the group consisting of

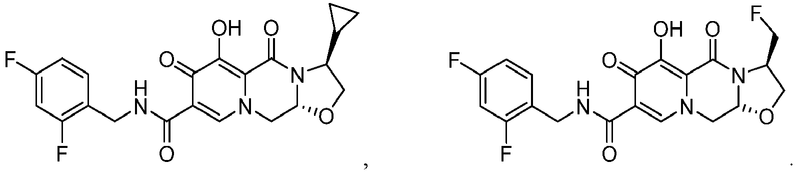

8. An isotope substitute of the compound according to any one of claims 1 to 7, preferably, the isotope substitute is deuterium atom substitute.

9. A pharmaceutical composition, comprising the compound or the pharmaceutically acceptable salt thereof according to any one of claims 1 to 8, and pharmaceutically acceptable excipient(s).

10. A method for treating or preventing HIV infection in a human, comprising a step of administering to the human a therapeutically effective amount of the compound or the pharmaceutically acceptable salt thereof according to any one of claims 1 to 8 or the pharmaceutical composition according to claim 9.

11. The compound or the pharmaceutically acceptable salt thereof according to any one of claims 1 to 8 or the pharmaceutical composition according to claim 9 for use in treating or preventing HIV infection in a human, wherein the compound or the pharmaceutically acceptable salt thereof or the pharmaceutical composition is administered in combination with one or more additional therapeutic agent(s).

12. The compound or the pharmaceutically acceptable salt thereof or the pharmaceutical composition according to claim 11, wherein the additional therapeutic agent is one or more selected from the group consisting of HIV protease inhibitor, non-nucleoside inhibitor of HIV reverse transcriptase, nucleoside inhibitor of HIV reverse transcriptase and nucleotide inhibitor of HIV reverse transcriptase.

13. The compound or the pharmaceutically acceptable salt thereof or the pharmaceutical composition according to claim 12, wherein the additional therapeutic agent is one or more selected from the group consisting of raltegravir, lamivudine, abacavir, ritonavir, dolutegravir, darunavir, atazanavir, emtricitabine, tenofovir, elvitegravir, rilpivirine and lopinavir.
